# EUROPÄISCHE PATENTSCHRIFT

(11) **EP 1 753 440 B1**
(45) Veröffentlichungstag und Bekanntmachung des Hinweises auf die Patenterteilung: **20.02.2008**
(21) Anmeldenummer: 05746643.5
(22) Anmeldetag: 04.05.2005
(51) Int. Cl.: A61K 35/74, A23L 1/03, A23L 1/30, A61K 9/36

(54) **ORALE DARREICHUNGSFORM ENTHALTEND PROBIOTISCHE BAKTERIEN**
ORAL FORM OF ADMINISTRATION CONTAINING PROBIOTIC BACTERIA
FORME POUR ADMINISTRATION ORALE, CONTENANT DES BACTERIES PROBIOTIQUES

(30) Priorität: 28.05.2004 DE 102004026706
(43) Veröffentlichungstag der Anmeldung: 21.02.2007
(73) Patentinhaber: Merck Patent GmbH, 64293 Darmstadt (DE)
(72) Erfinder: RUDOLPH, Markus, 1267 Vich (CH); HENKE, Stefan, 57548 Kirchen (DE); MANNECK, Iris, 64287 Darmstadt (DE)
(86) Internationale Anmeldenummer: PCT/EP2005/004835
(87) Internationale Veröffentlichungsnummer: WO 2005/117921

(56) Entgegenhaltungen:
- EP-A- 1 072 258
- DE-A1- 19 937 361
- PATENT ABSTRACTS OF JAPAN Bd. 016, Nr. 213 (C-0942), 20. Mai 1992 (1992-05-20) & JP 04 041434 A (ASAHI BREWERIES LTD), 12. Februar 1992 (1992-02-12)

## Beschreibung

Die Erfindung betrifft eine orale Darreichungsform, die wenigstens eine Gattung von probiotischen Mikroorganismen enthält, wobei sie selbst und/oder die probiotischen Bakterien mit einem Überzug versehen ist/sind, der mindestens zwei Celluloseether enthält.

Probiotische Mikroorganismen werden heute bereits vielfältig in Form von ausgesuchten Lebensmitteln, Nahrungsergänzungspräparaten oder Arzneimitteln eingesetzt, um Symptome, die eine gestörte oder geschädigte Darmflora hervorruft, zu lindern oder zu beseitigen. Ein Problem stellt der hohe Aktivitätsverlust der probiotischen Mikroorganismen von etwa 97% des Ausgangswertes bei oraler Einnahme am Ende des Dünndarms dar. Daher ist es notwendig eine wesentlich größere Menge der probiotischen Mikroorganismen bereitzustellen, um eine ausreichend hohe Aktivität zu erzielen.

DE 1937361 A1 beschreibt eine orale Darreichungsform mit probiotischen Mikroorganismen, in der der mit der Magen-Darm-Passage einhergehende Aktivitätsverlust der probiotischen Mikroorganismen durch Verwendung eines magensaftresistenten Überzuges bestehend aus Schellack verhindert werden soll. Nachteilig bei dieser Darreichungsform ist, dass die Auflösung des Überzugmaterials von mehreren physiologischen Bedingungen wie dem intraluminalen pH-Wert im Gastrointestinaltrakt, den Einnahmebedingungen (prä-, postprandial oder mit einer Mahlzeit), der Zusammensetzung der Mahlzeit, dem Alter des Anwenders, Krankheiten, Flüssigkeitsmenge und der gleichzeitigen Einnahme von Medikamenten, wie z. B. Antazida abhängig ist. Weiterhin ist die Verwendung und Verarbeitung von Schellack nicht unproblematisch, da Schellack ein Naturprodukt ist und infolge der damit verbundenen natürlichen Schwankungen in seiner Zusammensetzung nicht immer in der für eine reproduzierbares Auflösungsverhalten erforderlichen gleich bleibenden Qualität zur Verfügung steht. Bei erhöhter Feuchtigkeit kann es bei Schellack-Filmtabletten zu Verklebungen kommen, sodass die Integrität des Überzugs beeinträchtigt sein kann. Damit ist die Compliance des Anwenders und die Wirksamkeit der probiotischen Mikroorganismen möglicherweise nicht vollumfänglich sichergestellt. Weiterhin kann Schellack nur mit organischen Lösungsmitteln verarbeitet werden, was gegenüber der Verarbeitung von wässrigen Lösungen zu erhöhten Kosten führt sowie nachteilhaft zur Folge haben kann, dass in der Darreichungsform Reste an organischen Lösungsmitteln verbleiben, die aus toxikologischer Sicht unerwünscht sind. Darüber hinaus erfordert die Verwendung von Schellack als Überzugsmaterial höhere Mengen eines weichmachenden Zusatzes, da Schellack ohne Zusatz von Weichmachern infolge seiner sehr hohen Sprödigkeit und Brüchigkeit als Überzugsmaterial ungeeignet ist. Der Zusatz von Weichmachern ist aber problematisch, da diese während der Lagerung der fertigen Darreichungsform aus dem Schellackfilm in die Umgebung entweichen können, wodurch sich die Eigenschaften des Überzugs verschlechtern und die Lagerfähigkeit der Darreichungsform verkürzt wird. Hinzu kommt, dass Schellack während der Lagerung "altert", dass heißt die im Schellack enthaltenen funktionellen Gruppen miteinander reagieren können und somit quervernetzen, was zu einer Verlangsamung der Auflösungszeit des Schellacküberzugs führt.

Es war Aufgabe der vorliegenden Erfindung, eine orale Darreichungsform zur Verfügung zu stellen, weiche die probiotischen Mikroorganismen im menschlichen und/oder tierischen Darm reproduzierbar freisetzt, um die Aktivität der probiotischen Mikroorganismen und damit deren gesundheitsfördernde Wirkung im Darm sicherzustellen. Die orale Darreichungsform sollte weiterhin gut lagerfähig und einfach und preiswert herstellbar sein.

Die Aufgabe konnte überraschenderweise gelöst werden durch die Bereitstellung einer oralen Darreichungsform, die wenigstens eine Gattung von probiotischen Mikroorganismen enthält und die selbst und/oder in der die probiotischen Mikroorganismen mit einem Überzug, der mindestens zwei Celluloseether enthält, versehen ist/sind. Gegenstand der Erfindung ist daher eine orale Darreichungsform enthaltend wenigstens eine Gattung von probiotischen Mikroorganismen, die dadurch gekennzeichnet ist, dass sie selbst und/oder in der die probiotischen Mikroorganismen mit einem Überzug, der mindestens zwei Celluloseether enthält, versehen ist/sind.

Der Überzug aus mindestens zwei Celluloseethern kann aus wässrigen Lösungen aufgetragen werden, sodass Reste an organischen Lösungsmitteln grundsätzlich vermieden werden können. Vorteilhaft kann im Überzug auf den Zusatz von Weichmachern verzichtet werden, sodass die Lagerstabilität hierdurch nicht beeinträchtigt wird.

Vorzugsweise ist die orale Darreichungsform eine Tablette, ein Dragee, eine Kapsel, ein Granulat, eine Pelletzubereitung oder ein Pulver, besonders bevorzugt eine Tablette und ganz besonders bevorzugt eine Mehrschichttablette.

Als probiotische Mikroorganismen eignen sich alle Mikroorganismen, die im gesunden menschlichen oder tierischen Körper entweder selbst üblicherweise vorkommen und die eine gesundheitsfördernde Wirkung auf den gesunden, gestörten oder erkrankten menschlichen oder tierischen Körper haben.

Bevorzugt werden als probiotische Mikroorganismen lebende Lactobacillen, Bifidobakterien und/oder Streptokokken eingesetzt. Besonders bevorzugt sind die Spezies Lactobacillus casei, Lactobacillus acidophilus, Lactobacillus reuteri, Lactobacillus bifidum, Lactobacillus gasseri, Lactobacillus plantarum, Lactobacillus johnsonii, Lactobacillus rhamnosus, Lactobacillus fermentum, Lactobacillus paracasei, Lactobacillus crispatus, Bifidobakterium longum, Bifidobakterium bifidum, Bifidobakterium longum, Bifidobacterium lactis, Bifidobacterium brevis, Bifidobacterium animalis, Bifidobacterium adolescentis, Bifidobacterium infantis, Streptococcus thermophilus und/oder Lactococcus lactis.

Die Menge der probiotischen Mikroorganismen in der erfindungsgemäßen oralen Darreichungsform ist so zu wählen, daß die angestrebte gesundheitsfördernde Wirkung gewährleistet wird. Vorzugsweise enthält die erfindungsgemäße orale Darreichungsform 10³ bis 10¹², besonders bevorzugt 10⁵ bis 10¹¹ und ganz besonders bevorzugt 10⁷ bis 10¹⁰ probiotische Mikroorganismen. Für die Stabilität in Hinsicht auf die Anzahl und die Aktivität lebender Mikroorganismen ist es vorteilhaft, wenn die verwendeten Materialien, insbesondere das Trägermaterial, in welches die probiotischen Mikroorganismen eingebettet sind, einen möglichst geringen Wassergehalt aufweisen. Vorzugsweise beträgt der Wassergehalt ≤ 3,0 Gew.-%, besonders bevorzugt ≤ 0,1 Gew.-% bezogen auf das Gewicht des Trägermaterials.

Die im Überzug der erfindungsgemäßen oralen Darreichungsform enthaltenen Celluloseether sind in wässrigen Medien quellfähige bzw. gelbildende Stoffe, wobei die Quellung bzw. Gelbildung in Abhängigkeit von den im jeweiligen Celluloseether enthaltenen Ethersubstituenten unterschiedlich stark und mit unterschiedlicher Geschwindigkeit erfolgt. Der Überzug der erfindungsgemäßen oralen Darreichungsform enthält mindestens zwei Celluloseether mit jeweils unterschiedlichem Quellungs- bzw. Gelbildungsverhalten, die so aufeinander abgestimmt sind, dass die in der erfindungsgemäßen oralen Darreichungsform enthaltenen probiotischen Kulturen nach Einnahme der oralen Darreichungsform durch den Anwender infolge Quellung und letztendlich Auflösung und/oder strukturelle Ablösung des Überzugs zeitlich verzögert im Darm freigesetzt werden. Die zeitliche Verzögerung der Freisetzung ist weitgehend unabhängig von den jeweiligen pH-Verhältnissen und so bemessen, dass sie dem Zeitraum entspricht, die die orale Darreichungsform benötigt, um nach Einnahme durch den Anwender den Magen weitgehend unverändert zu passieren und in den Darm zu gelangen. Für die erfindungsgemäße orale Darreichungsform geeignet sind Verzögerungszeiten, welche die Überlebensrate der probiotischen Mikroorganismen gegenüber der nichtüberzogenen Darreichungsform im Dünndarm bis zum terminalen lleum um mindestens das 5-fache zu erhöhen. Die Dauer der Verzögerung ist abhängig von der Art der im Überzug enthaltenen Celluloseether und kann durch Variation der Mengenverhältnisse, in denen diese zueinander vorliegen, sowie durch Variation der Schichtdicke des Überzugs auf den gewünschten Wert eingestellt werden. Die für das gewünschte Freisetzungsprofil jeweils erforderlichen Mengenverhältnisse der Celluloseether zueinander sowie die jeweils erforderliche Schichtdicke können dabei anhand von Versuchen in *in-vitro* Modellen, beispielsweise dem sogenannten TNO-Modell (dynamic gastrointestinal model, wie beschrieben in Marteau, P et al. (1997) Survial of Lactic Acid Bacteria in a Dynamic Model of the Stomach and Small Inestine: Validation and the Effects of Bile, J Dairy Sci 80:1031-1037) ermittelt und optimiert werden.

Im allgemeinen enthält ein Überzug aus zwei verschiedenen Celluloseethern diese in einem Gewichtsverhältnis von 0,1:99,9 bis 99,9: 0,1. Die Schichtdicke des Überzugs beträgt im allgemeinen 0,5 bis 20 mg pro cm², bevorzugt 5 bis 15 mg pro cm².

Nach einer Ausführungsform der Erfindung enthält die orale Darreichungsform im Überzug Celluloseether, die als Ethersubstituenten Hydroxyalkylgruppen, bevorzugt Hydroxyethyl-, Hydroxypropyl- und/oder Dihydroxypropylgruppen, besonders bevorzugt Hydroxypropylgruppen, enthalten. Für die Erfindung einsetzbare Celluloseether mit Hydroxyalkylgruppen als Ethersubstituenten sind demgemäss z. B. Hydroxyethylcellulose, Hydroxypropylcellulose und Dihydroxypropylcellulose.

Nach einer bevorzugten Ausführungsform der Erfindung enthält mindestens einer der im Überzug der oralen Darreichungsform enthaltenen Celluloseether als Ethersubstituenten neben Hydroxyalkylgruppen auch Alkylgruppen, bevorzugt Methyl- und/oder Ethylgruppen, besonders bevorzugt Methylgruppen. Für die Erfindung einsetzbare Celluloseether, die als Ethersubstituenten neben Hydroxyalkylgruppen auch Alkylgruppen enthalten, sind beispielsweise Ethylhydroxyethylcellulose, Hydroxypropylmethylcellulose, Hydroxypropylethylcellulose und Hydroxyethylmethylcellulose.

Nach einer besonders bevorzugten Ausführungsform der Erfindung enthält der Überzug der erfindungsgemäßen oralen Darreichungsform einen Celluloseether, der als Ethersubstituenten ausschließlich Hydroxyalkylgruppen enthält, zusammen mit einem Celluloseether, der neben Hydroxyalkylgruppen auch Alkylgruppen als Ethersubstituenten enthält.

Ganz besonders bevorzugt enthält die erfindungsgemäße orale Darreichungsform im Überzug als Celluloseether Hydroxypropylmethylcellulose und Hydroxypropylcellulose. Hydroxypropylmethylcellulose und Hydroxypropylcellulose können dabei zueinander in einem Gewichtsverhältnis von 90:10 bis 10:90 vorliegen, bevorzugt liegen sie zueinander in einem Gewichtsverhältnis von 30:70 bis 70:30, besonders bevorzugt in einem Gewichtsverhältnis von ca. 35:65 vor. Vorzugsweise werden Hydroxypropylmethylcellulose und Hydroxypropylcellulose als binäres Gemisch eingesetzt.

Vorzugsweise beträgt der Gewichtsanteil der im Überzug enthaltenen Celluloseether, bezogen auf das Gesamtgewicht der oralen Darreichungsform, 1 bis 20 Gew.-%, besonders bevorzugt 1,5 bis 10 Gew.-% und ganz besonders bevorzugt 3 bis 5 Gew.-%.

Neben den erwähnten Celluloseethern kann der Überzug, beispielsweise zur Erhöhung seiner physikalischen Stabilität, auch Alkylcelluloseether enthalten, wie z. B. Methylcellulose oder Ethylcellulose. Soweit Alkylcelluloseether enthalten sind, sind diese, bezogen auf die Trockenmasse des Überzugs, vorzugsweise in einer Menge von 0,5 bis zu 10 Gew.-% enthalten.

Für die erfindungsgemäße orale Darreichungsform ist es wesentlich, daß sie von dem Überzug vollständig umschlossen ist.

Eine weitere bevorzugte Ausführungsform der oralen Darreichungsform enthält probiotische Mikroorganismen, die selbst mit einem magensaftresistenten Überzug versehen sind. Hierzu werden die probiotischen Mikroorganismen nach verschiedenen dem Fachmann bekannten Methoden getrocknet und anschließend mit dem Überzug versehen.

Bevorzugt enthält der Überzug keine weiteren Hilfsstoffe. Im Produktionsmaßstab kann es hilfreich sein ein Trennmittel einzusetzen. Bevorzugt werden dann Stearate, z.B. Magnesiumstearat, Glycerolmonostearat, Glyceryldipalmitostearat oder Talkum eingesetzt. Stearate können, bezogen auf die Trockenmasse des Überzugs, in einem Gewichtsanteil von 1 bis 10 Gew.-% enthalten sein, bevorzugt sind ca. 5 Gew.-%. Talkum kann bezogen auf die Trockenmasse des Überzugs in einem Gewichtsanteil von bis zu 100 Gew.-% enthalten seien, bevorzugt ist ein Gewichtsanteil von 30 bis 50 Gew.-%.

Der Überzug kann aus wässriger, organischer oder hydroalkoholischer Lösung aufgebracht werden. Bevorzugt wird der Überzug aus wässriger Lösung aufgebracht. Gegenstand der Erfindung ist daher auch ein Verfahren zur Herstellung der erfindungsgemäßen oralen Darreichungsform, das dadurch gekennzeichnet ist, dass der Überzug aus wässriger Lösung und/oder aus organischer Lösung, bevorzugt aus wässriger Lösung aufgebracht wird. Bei Auftrag des Überzugs mittels organischer Lösung erfolgt dieser vorzugsweise aus alkoholischer Lösung, besonders bevorzugt aus hydroalkoholischer Lösung, d. h. aus einer Mischung aus Wasser und Alkohol. Bevorzugt wird als Alkohol Ethanol eingesetzt.

Der Überzug kann nach den üblichen dem Fachmann bekannten Verfahren, wie z.B. Dragieren, Aufsprühen von Lösungen, Dispersionen oder Suspensionen, durch Schmelzverfahren oder durch Pulverauftragsverfähren aufgebracht werden. Bevorzugt erfolgt der Auftrag des Überzugs mittels eines Trommelcoaters oder im Wirbelschichtverfahren, beispielsweise nach dem Wurster-Verfahren.

Die Überzüge erscheinen klar bis opak. Zur Einfärbung können Farbpigmente, Lacke oder Farbstoffe zugesetzt werden.

Nach einer bevorzugten Ausführungsform enthält die erfindungsgemäße orale Darreichungsform zusätzlich zu den probiotischen Mikroorganismen weitere ernährungsrelevante Zusätze. Vorzugsweise enthält sie Vitamine, Mineralstoffe, Spurenelemente, Ballaststoffe, Enzyme, Pflanzenextrakte, Eiweiße, Kohlenhydrate und/oder Fette. Enthält die orale Darreichungsform ernährungsrelevante Zusätze, deren Verdauung bereits im Magen beginnt, wie z.B. Eiweiße, so ist es wichtig, dass diese ernährungsrelevanten Zusätze zumindest nicht vollständig dem Überzug umschlossen sind.

Hierbei kann es in Abhängigkeit von den eingesetzten ernährungsrelevanten Zusätzen notwendig sein, diese untereinander und/oder diese und die probiotischen Mikroorganismen so in die erfindungsgemäße orale Darreichungsform einzubringen, dass sie nicht miteinander in Kontakt kommen. Vorzugsweise wird dies durch das Einbringen der ernährungsrelevanten Zusätze und/oder Mikroorganismen in verschiedene Schichten einer Mehrschichttablette erreicht.

Bevorzugte Vitamine sind Vitamin A (β-Carotin), Vitamin C, Vitamin E, Vitamine des B-Komplexes und/oder Vitamin K. Besonders bevorzugte Vitamine sind Vitamin A, Vitamin C und/oder Vitamin E. Die Menge an den Vitaminen richtet sich in der Regel nach der empfohlenen Mindestbedarfdosis für das jeweilige Vitamin, wobei diese jedoch auch um durchschnittlich 50 bis 300 % überschritten werden kann. Bevorzugte Bereiche sind für das Vitamin C zwischen 50 und 300 mg, für das Vitamin E 10 bis 50 mg, für das Vitamin A ≤ 1,5 mg und für die Vitamine des B- Komplexes 10 µg bis 20 mg.

Bevorzugte Mineralstoffe sind für den Verzehr geeignete, anorganische oder organische Natrium-, Kalium-, Calcium, Magnesium-, Zink- und/oder Eisensalze, welche vorzugsweise als Carbonate, Bicarbonate, Phosphate, Biphosphate, Sulfate, Bisulfate, Chloride, Fluoride, Citrate und/oder Lactate vorliegen. Der Anteil an Mineralstoffen bezogen auf das Gesamtgewicht der oralen Darreichungsform beträgt vorzugsweise von 20 bis 40 Gew.-%. Vorzugsweise enthält die erfindungsgemäße orale Darreichungsform als Spurenelemente Silicium, Chrom, Mangan, Jod, Molybdän und/oder Selen.

Als Ballaststoffe enthält die erfindungsgemäße orale Darreichungsform vorzugsweise Sojakleie, Maiskleie, Weizenkleie und/oder Getreideschrot, besonders bevorzugt Sojakleie. Der Anteil an Ballaststoffen bezogen auf das Gesamtgewicht der oralen Darreichungsform beträgt vorzugsweise 2 bis 50 Gew.-%.

Bevorzugte Enzyme bzw. Co-Enzyme sind Lipasen und/oder Proteasen bzw. CoEnzym Q, Superoxiddismutase und/oder Gluthathionperoxidase, die die Magen und/oder Darmfunktion und/oder den Stoffwechsel fördern. Diese können in an sich bekannter Menge und in an sich bekannter Form eingebracht werden.

Die orale Darreichungsform kann außerdem weitere probiotische Substanzen, vorzugsweise Oligo-Fructose und/oder andere Oligozucker enthalten.

Als Pflanzenextrakte bevorzugt sind Trockenextrakte und hier insbesondere solche, die Bioflavonoide, Polyphenole, Phytoöstrogene und/oder Saponine enthalten, wie z.B. aus Echinaceae.

Vorzugsweise enthält die erfindungsgemäße orale Darreichungsform als Eiweiße Sojaprotein und/oder Molkenprotein und/oder als Fette solche Fette, die mehrfach ungesättigte Fettsäuren enthalten.

Die erfindungsgemäße orale Darreichungsform kann außerdem je nach Ausführungsform übliche Hilfs- und Zusatzstoffe enthalten. Die Auswahl der Hilfs- und/oder Zusatzstoffe hängt auch von den lebensmittelrechtlichen Bestimmungen des Landes ab, in dem die erfindungsgemäße orale Darreichungsform zum Einsatz kommen soll. Als Hilfs- und/oder Zusatzstoffe kommen, beispielsweise für die erfindungsgemäßen Tabletten, Mehrschichttabletten und/oder Dragees, Stärke (z.B. Maisstärke), Talkum, mikrokristalline Cellulose, Lactose, hochdisperses Siliciumdioxid, Polyvinylpyrrolidon und/oder Cellulosepulver zum Einsatz. Als weitere Bestandteile können als Bindemittel und/oder Trennmittel Kohlenhydrate, wie beispielsweise Mannit, Sorbit, Xylit, Glucose, Sucrose, Fructose, Maltose, Dextrose, Maltodextrin und/oder Kaolin und/oder Cellulosederivate, wie beispielsweise Methylcellulose, Hydroxypropylcellulose und/oder Hydroxypropylmethylcellulose und/oder Calciumcarbonat, Calcium-, Magnesium und/oder Glycerinstearat eingesetzt werden. Des weiteren kann die erfindungsgemäße orale Darreichungsform auch Farb-, Geschmacks- und/oder Aromastoffe, sowie Gleitmittel, Antioxidantien und/oder Stabilisatoren enthalten. Der Gehalt dieser Grundlagenstoffe richtet sich einerseits nach dem angestrebten Gehalt an probiotischen Mikroorganismen, Vitaminen, Enzymen, Ballaststoffen u.s.w. und andererseits nach Kriterien, die die mechanischphysikalischen Eigenschaften der oralen Darreichungsform bestimmen, wie z.B. Härte, Verpressbarkeit, Größe, Farbe und/oder Form.

Die Herstellung der erfindungsgemäßen oralen Darreichungsform kann nach verschiedenen dem Fachmann bekannten Methoden durchgeführt werden. Diese Methoden sind z.B. aus H. Sucker, P. Fuchs, P. Speiser, "Pharmazeutische Technologie", Stuttgart 1978 oder K.H. Bauer, K.H. Frömming, C. Führer, "Pharmazeutische Technologie", Stuttgart 1986 bekannt.

Die Beispiele, ohne hierauf beschränkt zu sein, erläutern die Erfindung.

### Beispiel 1

Auf einer Rundläufer Tablettenpresse der Firma E. Hata wurde eine Mischung aus 45% Bakterienzubereitung, 28,7% Tricalciumphosphat, 19% mikrokristalliner Cellulose, 2% Glycerylpalmitostearat, 0,6% Magnesiumstearat und 4,7% Sprengmittel zusammen mit einer Vitamin- und Mineralstoffmischung zu einer bohnenförmigen Tablette mit einen Kerngewicht von 1,0 g und den Maßen 18,0 mm x 8,0 mm x 7,2 mm verpresst. Im Anschluss daran wurde eine Mischung aus 35 Teilen Hydroxypropylmethylcellulose und 65 Teilen Hydroxypropylcelluose aus wässriger Lösung in einem Trommelcoater der Firma O'Hara mit einer Chargengröße von 15 kg aufgesprüht. Die Menge an aufgesprühter Mischung Hydroxypropylmethylcellulose/Hydroxypropylcelluose betrug 5 Gew.-% bezogen auf das Gewicht des Kernes, entsprechend 11,74 mg pro cm² Tablettenoberfläche.

In einem in-vitro Versuch im TNO Modell werden die nicht-überzogenen Tablettenkerne mit den überzogenen Tabletten aus Beispiel 1 verglichen. Die Wiederfindung vom 100% Ausgangswert stellt die Überlebensrate der probiotischen Mikroorganismen nach Passage durch das Modell des Magen und Dünndarms dar.

| | Überlebensrate Bifidobacterien | |
|---|---|---|
| Passagezeit (min) | Tablettenkern | Überzogene Tablette |
| 360 | 2,3 % | 21,2 % |

### Beispiel 2

Auf einer Rundläufer-Tablettenpresse der Firma Fette wurde eine Mischung aus 65% Bakterienzubereitung, 20% Tricalciumphosphat, 6% mikrokristalliner Cellulose, 2% Glycerylpalmitostearat, 0,6% Magnesiumstearat und 6,4 % Sprengmittel zusammen mit einer Vitamin- und Mineralstoffmischung zu einer bohnenförmigen Tablette mit einem Kerngewicht von 1,35 g und den Maßen 21,0 mm x 10,0 mm x 8 mm verpresst. Im Anschluss daran wurde eine Mischung aus 50 Teilen Hydroxypropylmethylcellulose und 50 Teilen Hydroxypropylcelluose aus wässriger Lösung in einem Trommelcoater der Firma O'Hara mit einer Batchgröße von 15 kg aufgesprüht. Die Menge an aufgesprühter Mischung Hydroxypropylmethylcellulose/Hydroxypropylcelluose betrug 7 Gew.-% bezogen auf das Gewicht des Kernes, entsprechend 17,42 mg pro cm² Tablettenoberfläche.

### Beispiel 3

Auf einer Rundläufer-Tablettenpresse der Firma E. Hata wurde eine Mischung aus 45% Bakterienzubereitung, 28,7% Tricalciumphosphat, 19% mikrokristalliner Cellulose, 2% Glycerylpalmitostearat, 0,6% Magnesiumstearat und 4,7% Sprengmittel zusammen mit einer Vitamin- und Mineralstoffmischung zu einer bohnenförmigen Tablette mit einem Kerngewicht von 1,0 g und den Maßen 18,0 mm x 8,0 mm x 7,2 mm verpresst. Im Anschluss daran wurde eine Mischung aus 35 Teilen Hydroxypropylmethylcellulose und 65 Teilen Hydroxypropylcelluose aus wässriger Lösung in einem Trommelcoater der Firma Pellegrini mit einer Bauchgröße von 250 kg aufgesprüht. Die Menge an aufgesprühter Mischung Hydroxypropylmethylcellulose/Hydroxypropylcelluose betrug 5 Gew.-% bezogen auf das Gewicht des Kernes, entsprechend 11,74 mg pro cm² Tablettenoberfläche.

In einem in-vitro Versuch im TNO Modell werden die nicht-überzogenen Tablettenkerne mit den überzogenen Tabletten aus Beispiel 3 verglichen. Die Wiederfindung vom 100% Ausgangswert stellt die Überlebensrate der probiotischen Mikroorganismen nach Passage durch das Modell des Magen und Dünndarms dar.

| | Überlebensrate Lactobacillus | |
|---|---|---|
| Passagezeit (min) | Tablettenkern | Überzogene Tablette |
| 360 | 1,6 % | 9,7 % |

### Beispiel 4

Auf einer Rundläufer-Tablettenpresse der Firma Fette wurde eine Mischung aus 65% Bakterienzubereitung, 20% Tricalciumphosphat, 6% mikrokristalliner Cellulose, 2% Glycerylpalmitostearat, 0,6% Magnesiumstearat und 6,4 % Sprengmittel zusammen mit einer Vitamin- und eine Mineralstoffmischung zu einer bohnenförmigen Tablette mit einem Kerngewicht von 1,35 g und den Maßen 21,0 mm x 10,0 mm x 8 mm verpresst. Im Anschluss daran wurde eine Mischung aus 35 Teilen Hydroxypropylmethylcellulose und 65 Teilen Hydroxypropylcelluose aus wässriger Lösung in einem Trommelcoater der Firma O'Hara mit einer Bauchgröße von 15 kg aufgesprüht. Die Menge an aufgesprühter Mischung Hydroxypropylmethylcellulose/Hydroxypropylcelluose betrug 7 Gew.-% bezogen auf das Gewicht des Kernes, entsprechend 17,42 mg pro cm² Tablettenoberfläche.

### Beispiel 5

Auf einer Rundläufer-Tablettenpresse der Firma E. Hata wurde eine Mischung aus 45% Bakterienzubereitung, 28,7% Tricalciumphosphat, 19% mikrokristalliner Cellulose, 2% Glycerylpalmitostearat, 0,6% Magnesiumstearat und 4,7% Sprengmittel zusammen mit einer Vitamin- und eine Mineralstoffmischung zu einer bohnenförmigen Tablette mit einem Kerngewicht von 1,0 g und den Maßen 18,0 mm x 8,0 mm x 7,2 mm verpresst. Im Anschluss daran wurde eine Mischung aus 35 Teilen Hydroxypropylmethylcellulose und 65 Teilen Hydroxypropylcelluose in wässrige Lösung gebracht und anschließend Magnesiumstearat mit 5 Gew.-% bezogen auf die Polymertrockensubstanz eingearbeitet und in einem Trommelcoater der Firma Pellegrini mit einer Bauchgröße von 250 kg aufgesprüht. Die Menge an aufgesprühter Mischung Hydroxypropylmethylcellulose/Hydroxypropylcelluose betrug 5 Gew.-% bezogen auf das Gewicht des Kernes, entsprechend 11,74 mg pro cm² Tablettenoberfläche.

### Beispiel 6

Auf einer Rundläufer-Tablettenpresse der Firma E. Hata wurde eine Mischung aus 45% Bakterienzubereitung, 28,7% Tricalciumphosphat, 19% mikrokristalliner Cellulose, 2% Glycerylpalmitostearat, 0,6% Magnesiumstearat und 4,7% Sprengmittel zusammen mit einer Vitamin- und eine Mineralstoffmischung zu einer bohnenförmigen Tablette mit einem Kerngewicht von 1,0 g und den Maßen 18,0 mm x 8,0 mm x 7,2 mm verpresst. Im Anschluss daran wurde eine Mischung aus 35 Teilen Hydroxypropylmethylcellulose und 65 Teilen Hydroxypropylcelluose in einer Ethanol-Wasser-Mischung (70Teile:30Teile) in Lösung gebracht und anschließend Magnesiumstearat mit 5 Gew.-% bezogen auf die Polymertrockensubstanz eingearbeitet und in einem Trommelcoater der Firma Pellegrini mit einer Bauchgröße von 333 kg aufgesprüht. Die Menge an aufgesprühter Mischung Hydroxypropylmethylcellulose/Hydroxypropylcelluose betrug 7 Gew.-% bezogen auf das Gewicht des Kernes, entsprechend 16,43 mg pro cm² Tablettenoberfläche.

### Beispiel 7

Auf einer Rundläufer Tablettenpresse der Firma E. Hata wurde eine Mischung aus 45% Bakterienzubereitung, 28,7% Tricalciumphosphat, 19% mikrokristalliner Cellulose, 2% Glycerylpalmitostearat, 0,6% Magnesiumstearat und 4,7% Sprengmittel zusammen mit einer Vitamin- und Mineralstoffmischung zu einer bohnenförmigen Tablette mit einen Kemgewicht von 1,0 g und den Maßen 18,0 mm x 8,0 mm x 7,2 mm verpresst. Im Anschluss daran wurde eine Mischung aus 35 Teilen Hydroxypropylmethylcellulose und 60 Teilen Hydroxypropylcelluose und 5 Teilen Hydroxyethylcellulose aus wässriger Lösung in einem Trommelcoater der Firma Bohle mit einer Chargengröße von 5 kg aufgesprüht. Die Menge an aufgesprühter Mischung Hydroxypropylmethylcellulose/Hydroxypropylcelluose/Hydroxyethylcellulose betrug 5 Gew.-% bezogen auf das Gewicht des Kernes, entsprechend 11,74 mg pro cm² Tablettenoberfläche.

### Beispiel 8

Auf einer Rundläufer-Tablettenpresse der Firma E. Hata wurde eine Mischung aus 9,8% Bakterienzubereitung, 35,0% Inulin, 28,7% Tricalciumphosphat, 18,9% mikrokristalliner Cellulose, 2,0% Glycerylpalmitostearat, 0,6% Magnesiumstearat und 5,0% Sprengmittel zusammen mit einer Vitamin- und eine Mineralstoffmischung zu einer bohnenförmigen Tablette mit einem Kemgewicht von 1,0 g und den Maßen 18,0 mm x 8,0 mm x 7,2 mm verpresst. Im Anschluss daran wurde eine Mischung aus 65 Teilen Hydroxypropylmethylcellulose und 35 Teilen Hydroxypropylcelluose in wässrige Lösung gebracht und anschließend Magnesiumstearat mit 5 Gew.-% bezogen auf die Polymertrockensubstanz eingearbeitet und in einem Trommelcoater der Firma Pellegrini mit einer Batchgröße von 333 kg aufgesprüht. Die Menge an aufgesprühter Mischung Hydroxypropylmethylcellulose/Hydroxypropylcelluose betrug 5 Gew.-% bezogen auf das Gewicht des Kernes, entsprechend 11,74 mg pro cm² Tablettenoberfläche.

## Patentansprüche

1. Orale Darreichungsform enthaltend wenigstens eine Gattung von probiotischen Mikroorganismen, **dadurch gekennzeichnet, dass** sie selbst und/oder die probiotischen Mikroorganismen mit einem Überzug versehen ist/sind, der mindestens zwei Celluloseether enthält.

2. Orale Darreichungsform gemäß Anspruch 1, **dadurch gekennzeichnet, dass** sie eine Tablette, ein Dragee, eine Kapsel, ein Granulat oder ein Pulver, vorzugsweise eine Tablette und besonders bevorzugt eine Mehrschichttablette ist.

3. Orale Darreichungsform nach Anspruch 1 oder 2, **dadurch gekennzeichnet, dass** die probiotischen Mikroorganismen Lactobacillen, Bifidobakterien, oder Streptokokken, vorzugsweise Lactobacillus casei, Lactobacillus acidophilus, Lactobacillus reuteri, Lactobacillus bifidum, Lactobacillus gasseri, Lactobacillus plantarum, Lactobacillus johnsonii, Lactobacillus rhamnosus, Lactobacillus fermentum, Lactobacillus paracasei, Lactobacillus crispatus, Bifidobakterium longum, Bifidobakterium bifidum, Bifidobakterium longum, Bifidobacterium lactis, Bifidobacterium brevis, Bifidobacterium animalis, Bifidobacterium adolescentis, Bifidobacterium infantis, Streptococcus thermophilus und/oder Lactococcus lactis sind.

4. Orale Darreichungsform nach einem oder mehreren der Ansprüche 1 bis 3, **dadurch gekennzeichnet, dass** sie 10³ bis 10¹² vorzugsweise 10⁵ bis 10¹¹ und besonders bevorzugt 10⁷ bis 10¹⁰ probiotische Mikroorganismen enthält.

5. Orale Darreichungsform nach einem oder mehreren der Ansprüche 1 bis 4, **dadurch gekennzeichnet, dass** die im Überzug enthaltenen Celluloseether als Ethersubstituenten Hydroxyalkylgruppen, bevorzugt Hydroxyethyl-, Hydroxypropyl- und/oder Dihydroxypropylgruppen, besonders bevorzugt Hydroxypropylgruppen, enthalten.

6. Orale Darreichungsform nach Anspruch 5, **dadurch gekennzeichnet, dass** mindestens einer der im Überzug enthaltenen Celluloseether als Ethersubstituenten neben Hydroxyalkylgruppen auch Alkylgruppen, bevorzugt Methyl- und/oder Ethylgruppen, besonders bevorzugt Methylgruppen enthält.

7. Orale Darreichungsform nach Anspruch 6, **dadurch gekennzeichnet, dass** der Überzug einen Celluloseether, der als Ethersubstituenten ausschließlich Hydroxyalkylgruppen enthält, zusammen mit einem Celluloseether, der als Ethersubstituenten neben Hydroxyalkylgruppen auch Alkylgruppen enthält, enthält.

8. Orale Darreichungsform nach Anspruch 7, **dadurch gekennzeichnet, dass** als Celluloseether Hydroxyproylmethylcellulose und Hydroxypropylcellulose enthalten sind.

9. Orale Darreichungsform nach Anspruch 8, **dadurch gekennzeichnet, dass** die Hydroxyproylmethylcellulose und die Hydroxypropylcellulose zueinander in einem Gewichtsverhältnis von 90:10 bis 10:90, bevorzugt in einem Gewichtsverhältnis von 70:30 bis 30:70, besonders bevorzugt in einem Gewichtsverhältnis von ca. 35:65 vorliegen.

10. Orale Darreichungsform nach einem oder mehreren der Ansprüche 1 bis 8, **dadurch gekennzeichnet, dass** sie weitere ernährungsrelevante Zusätze, vorzugsweise Vitamine, Mineralstoffe, Spurenelemente, Ballaststoffe, Enzyme, Pflanzenextrakte, Eiweiße, Kohlenhydrate und/oder Fette enthält.

11. Verfahren zur Herstellung einer oralen Darreichungsform nach einem oder mehreren der Ansprüche 1 bis 9, **dadurch gekennzeichnet, dass** der Überzug aus wässriger Lösung und/oder aus organischer Lösung, bevorzugt aus wässriger Lösung aufgebracht wird.

## Claims

1. Oral administration form comprising at least one species of probiotic microorganisms, **characterised in that** it itself and/or the probiotic microorganisms is/are provided with a coating which comprises at least two cellulose ethers.

2. Oral administration form according to Claim 1, **characterised in that** it is a tablet, a dragee, a capsule, a granular material or a powder, preferably a tablet and particularly preferably a multilayered tablet.

3. Oral administration form according to Claim 1 or 2, **characterised in that** the probiotic microorganisms are lactobacilli, bifidobacteria or streptococci, preferably Lactobacillus casei, Lactobacillus acidophilus, Lactobacillus reuteri, Lactobacillus bifidum, Lactobacillus gasseri, Lactobacillus plantarum, Lactobacillus johnsonii, Lactobacillus rhamnosus, Lactobacillus fermentum, Lactobacillus paracasei, Lactobacillus crispatus, Bifidobacterium longum, Bifidobacterium bifidum, Bifidobacterium longum, Bifidobacterium lactis, Bifidobacterium brevis, Bifidobacterium animalis, Bifidobacterium adolescentis, Bifidobacterium infantis, Streptococcus thermophilus and/or Lactococcus lactis.

4. Oral administration form according to one or more of Claims 1 to 3, **characterised in that** it comprises 10³ to 10¹², preferably 10⁵ to 10¹¹ and particularly preferably 10⁷ to 10¹⁰ probiotic microorganisms.

5. Oral administration form according to one or more of Claims 1 to 4, **characterised in that** the cellulose ethers present in the coating contain hydroxyalkyl groups, preferably hydroxyethyl, hydroxypropyl and/or dihydroxypropyl groups, particularly preferably hydroxypropyl groups, as ether substituents.

6. Oral administration form according to Claim 5, **characterised in that** at least one of the cellulose ethers present in the coating also contains alkyl groups, preferably methyl and/or ethyl groups, particularly preferably methyl groups, as ether substituents besides hydroxyalkyl groups.

7. Oral administration form according to Claim 6, **characterised in that** the coating comprises a cellulose ether, which contains exclusively hydroxyalkyl groups as ether substituents, together with a cellulose ether, which also contains alkyl groups as ether substituents besides hydroxyalkyl groups.

8. Oral administration form according to Claim 7, **characterised in that** the cellulose ethers present are hydroxypropylmethylcellulose and hydroxypropylcellulose.

9. Oral administration form according to Claim 8, **characterised in that** the hydroxypropylmethylcellulose and the hydroxypropylcellulose are present in a weight ratio to one another of 90:10 to 10:90, preferably in a weight ratio of 70:30 to 30:70, particularly preferably in a weight ratio of about 35:65.

10. Oral administration form according to one or more of Claims 1 to 8, **characterised in that** it comprises further nutrition-relevant additives, preferably vitamins, mineral substances, trace elements, roughage, enzymes, plant extracts, proteins, carbohydrates and/or fats.

11. Process for the production of an oral administration form according to one or more of Claims 1 to 9, **characterised in that** the coating is applied from aqueous solution and/or from organic solution, preferably from aqueous solution.

## Revendications

1. Forme d'administration orale comprenant au moins une espèce de microorganismes probiotiques, **caractérisée en ce qu'**elle-même et/ou les microorganismes probiotiques est/sont muni(e)(s) d'un revêtement qui comprend au moins deux éthers de cellulose.

2. Forme d'administration orale selon la revendication 1, **caractérisée en ce qu'**il s'agit d'un comprimé, d'une dragée, d'une gélule, d'un matériau granulaire ou d'une poudre, de préférence un comprimé et de façon particulièrement préférable, un comprimé multicouche.

3. Forme d'administration orale selon la revendication 1 ou 2, **caractérisée en ce que** les microorganismes probiotiques sont des lactobacilles, des bifidobactéries ou des streptocoques, de préférence Lactobacillus casei, Lactobacillus acidophilus, Lactobacillus reuteri, Lactobacillus bifidum, Lactobacillus gasseri, Lactobacillus plantarum, Lactobacillus johnsonii, Lactobacillus rhamnosus, Lactobacillus fermentum, Lactobacillus paracasei, Lactobacillus crispatus, Bifidobacterium longum, Bifidobacterium bifidum, Bifidobacterium longum, Bifidobacterium lactis, Bifidobacterium brevis, Bifidobacterium animalis, Bifidobacterium adolescentis, Bifidobacterium infantis, Streptococcus thermophilus et/ou Lactococcus lactis.

4. Forme d'administration orale selon une ou plusieurs des revendications 1 à 3, **caractérisée en ce qu'**elle comprend 10³ à 10¹², de préférence 10⁵ à 10¹¹ et de façon particulièrement préférable, 10⁷ à 10¹⁰ microorganismes probiotiques.

5. Forme d'administration orale selon une ou plusieurs des revendications 1 à 4, **caractérisée en ce que** les éthers de cellulose présents dans le revêtement contiennent des groupes hydroxyalkyle, de préférence des groupes hydroxy-éthyle, hydroxypropyle et/ou dihydroxypropyle, de façon particulièrement préférable, des groupes hydroxypropyle, en tant que substituants d'éther.

6. Forme d'administration orale selon la revendication 5, **caractérisée en ce qu'**au moins l'un des éthers de cellulose présents dans le revêtement contient également des groupes alkyle, de préférence des groupes méthyle et/ou éthyle, de façon particulièrement préférable, des groupes méthyle, en tant que substituants d'éther en plus de groupes hydroxyalkyle.

7. Forme d'administration orale selon la revendication 6, **caractérisée en ce que** le revêtement comprend un éther de cellulose qui contient exclusivement des groupes hydroxyalkyle en tant que substituants d'éther en association avec un éther de cellulose qui contient également des groupes alkyle en tant que substituants d'éther plus de groupes hydroxyalkyle.

8. Forme d'administration orale selon la revendication 7, **caractérisée en ce que** les éthers de cellulose présents sont hydroxypropylméthylcellulose et hydroxypropylcellulose.

9. Forme d'administration orale selon la revendication 8, **caractérisée en ce que** l'hydroxypropylméthylcellulose et l'hydroxypropylcellulose sont présents selon un rapport en poids de l'un sur l'autre de 90:10 à 10:90, de préférence selon un rapport en poids de 70:30 à 30:70, de façon particulièrement préférable, selon un rapport en poids d'environ 35:65.

10. Forme d'administration orale selon une ou plusieurs des revendications 1 à 8, **caractérisée en ce qu'**elle comprend en outre des additifs concernant la nutrition, de préférence des vitamines, des substances minérales, des oligo-éléments, des aliments de lest, des enzymes, des extraits de plante, des protéines, des hydrates de carbone et/ou des matières grasses.

11. Procédé pour la production d'une forme d'administration orale selon une ou plusieurs des revendications 1 à 9, **caractérisé en ce que** le revêtement est appliqué à partir d'une solution aqueuse et/ou à partir d'une solution organique, de préférence à partir d'une solution aqueuse.
